# EUROPÄISCHE PATENTSCHRIFT

(11) **EP 0 683 766 B1**
(45) Veröffentlichungstag und Bekanntmachung des Hinweises auf die Patenterteilung: **09.07.1997**
(21) Anmeldenummer: 94907546.9
(22) Anmeldetag: 11.02.1994
(51) Int. Cl.: C07C 227/08, C07C 231/12, C07C 229/12, C07C 233/36, C07C 69/003, C11D 1/94

(54) **VERFAHREN ZUR HERSTELLUNG WASSERFREIER DETERGENSGEMISCHE**
PROCESS FOR PREPARING WATER-FREE DETERGENT MIXTURES
PROCEDE DE FABRICATION DE MELANGES DETERGENTS ANHYDRES

(30) Priorität: 19.02.1993 DE 4305083
(43) Veröffentlichungstag der Anmeldung: 29.11.1995
(73) Patentinhaber: Henkel Kommanditgesellschaft auf Aktien, 40191 Düsseldorf (DE)
(72) Erfinder: PI, Raphael, E-08400 Granollers (ES); BIGORRA-LOSAS, Joaquim, E-08202 Sadabell (ES); PONSATI-OBIOLS, Oriol, E-08025 Barcelona (ES); SCHMID, Karl, D-40822 Mettmann (DE)
(86) Internationale Anmeldenummer: EP9400392
(87) Internationale Veröffentlichungsnummer: WO9419313

(56) Entgegenhaltungen:
- EP-A- 0 243 619
- EP-A- 0 353 580
- DE-A- 4 207 386

## Beschreibung

### Gebiet der Erfindung

Die Erfindung betrifft ein Verfahren zur Herstellung wasserfreier Detergensgemische, bei dem man Amine in Gegenwart von nichtionischen Tensiden alkyliert, die Verfahrensprodukte sowie deren Verwendung in oberflächenaktiven Mitteln.

### Stand der Technik

Amphotere bzw. zwitterionische Tenside sind ausgesprochen hautverträglich und weisen ausgezeichnete Reinigungseigenschaften auf. Sie eignen sich daher in besonderer Weise zur Konfektionierung einer Vielzahl von oberflächenaktiven Produkten. Zu ihrer Herstellung geht man im einfachsten Fall von sekundären oder tertiären Aminen aus, die mit Natriumchloracetat zu Alkylbetainen umgesetzt werden. Die Umsetzung von Fettsäureamidoaminen oder Imidazolinen mit Natriumchloracetat führt zur Bildung von amphoteren Tensiden vom Typ der Glycinate, wird als Alkylierungsmittel Acrylsäureester eingesetzt, bilden sich Aminopropionate. Verbindungen der genannten Art sind in einer Vielzahl von Übersichtsartikeln beschrieben, von denen an dieser Stelle nur **Parf.Cosm.Arom. 70, 67 (1986), HAPPI, 70, (Nov.1986)** und **Soap Cosm.Chem.Spec. 46, (Apr.1990)** genannt sein sollen.

Ein besonderes Anliegen bei der Herstellung der amphoteren bzw. zwitterionischen Tenside besteht darin, möglichst hochkonzentrierte, jedoch fließfähige Produkte herzustellen, um auf diesem Wege Transport- und Lagerkosten zu minimieren. Des weiteren ist es erforderlich, die an sich ausgezeichnete Hautverträglichkeit der Produkte nicht durch Spuren an nicht umgesetzten Alkylierungsmittel zu beeinträchtigen und eine Phasentrennung infolge eines zu hohen Elektrolytgehaltes zu vermeiden.

In der Vergangenheit hat es nicht an Vorschlägen gemangelt, eines oder mehrere der genannten Probleme zu lösen. So ist aus der **DE-A 29 26 479** (Goldschmidt) ein Verfahren zur Herstellung von Alkylamidobetainen bekannt, bei dem man den Restgehalt an Alkylierungsmittel minimiert, indem man die Reaktion bei einem pH-Wert von 7,5 bis 10,5 durchführt. In die gleiche Richtung weist die Lehre der **DE-A 20 63 424** (Rewo), die die pH-Regulierung für die Alkylierung von Imidazolinen beschreibt.

Gemäß der **DE-A1 40 40 887** (Goldschmidt) wird zur Herstellung von fließfähigen wäßrigen Betaindispersionen vorgeschlagen, die Quaternierung in wäßriger oder alkoholischer Lösung unter Zusatz anionischer Tenside durchzuführen. Aus der **GB-A 20 22 125** ist ferner bekannt, daß sich C_{12/14}-Kokosalkyldimethylamin in Gegenwart einer wäßrigen Lösung von Natriumlaurylsulfat mit Natriumchloracetat alkylieren läßt. In beiden Schriften wird jedoch ausdrücklich darauf hingewiesen, daß die Anwesenheit von Wasser während der Alkylierung unbedingt erforderlich ist, da andernfalls nicht mehr fließfähige Produkte mit einer lamellaren Flüssigkristallstruktur entstehen würden. Nach den genannten Verfahren können somit zwar hochkonzentrierte, jedoch keine wasserfreien Tensidpasten erhalten werden.

Ein weiteres Verfahren zur Herstellung von fließ- und pumpfähigen Betainen mit einem Aktivsubstanzgehalt von mindestens 70 Gew.-% ist aus der **EP-B1 0 243 619** (Goldschmidt) bekannt. Hierin wird vorgeschlagen, als Ausgangsstoffe ausschließlich solche Amidoamine einzusetzen, die einen Schmelzpunkt von maximal 30°C aufweisen und die Quaternierung mit Kalium- bzw. Ammoniumchloracetat in einem organischen Lösungsmittel, das höchstens 20 Gew.-% Wasser enthalten darf, durchzuführen. Für eine technische Realisierung kommt ein solches Verfahren indes wohl kaum in Betracht, da die erforderliche Abtrennung des Lösungsmittels vom Wertprodukt nach Abschluß der Alkylierung mit einem erheblichen technischen Aufwand verbunden wäre.

Aus der **EP-B1 0 302 329** (Goldschmidt) ist ferner bekannt, daß sich fließfähige Betainpasten mit einem Gehalt von ca. 40 Gew.-% herstellen lassen, indem man die nach konventionellen Quaternierungsverfahren erhaltenen wäßrigen Betainlösungen auf den gewünschten Wassergehalt eindampft und den pH-Wert anschließend durch Zugabe von Mineralsäure auf 1 bis 4,5 einstellt.

In der **EP-A2 0 353 580** (Goldschmidt) wird vorgeschlagen, konzentrierte, fließfähige wäßrige, gegebenenfalls niedere aliphatische Alkohole enthaltende Lösungen von Betainen herzustellen, in dem man die Quaternierung in wäßriger oder wäßrig-alkoholischer Lösung unter Zusatz einer solchen Menge nichtionischer Tenside durchführt, daß die resultierende Lösung einen Niotensidgehalt von vorzugsweise 3 bis 20 Gew.-% aufweisen. Als geeignete nichtionische Tenside kommen hierbei vor allem Fettsäurepolyethylenoxidester in Betracht. Als weitere geeignete - jedoch ausdrücklich nicht bevorzugte - Tenside werden Fettalkoholalkoxylate mit einem HLB-Wert im Bereich von 14 bis 20 genannt. Die Lehre dieser Druckschrift geht jedoch dahin, die Quaternierung in Gegenwart von Wasser und/oder Alkoholen - vorzugsweise Ethanol - durchzuführen und zur Viskositätserniedrigung den Betainen vor der Quaternierung, vorzugsweise jedoch im Anschluß daran, äußerst hydrophile nichtionische Tenside, beispielsweise Polysorbate mit 120 Ethylenoxid-Einheiten, zuzusetzen. Auch nach diesem Verfahren lassen sich somit keine wasserfreien Produkte herstellen. Ein weiterer Nachteil besteht darin, daß zwangsläufig Compounds von Betainen und äußerst hydrophilen nichtionischen Tensiden erhalten werden, wobei letztere aufgrund ihrer mangelnden Schaumentwicklung für kosmetische Produkte oder Geschirrspülmittel wenig geeignet sind.

Die Aufgabe der Erfindung hat somit darin bestanden, amphotere Tenside in wasserfreier Form zur Verfügung zu stellen und dabei die Nachteile des Stands der Technik zu vermeiden.

### Beschreibung der Erfindung

Gegenstand der Erfindung ist ein Verfahren zur Herstellung wasserfreier Detergensgemische, bei dem man sekundäre oder tertiäre Amine in Gegenwart von Fettalkoholpolyglycolethern mit einem HLB-Wert im Bereich von 6 bis 12, jedoch ohne Zusatz von Wasser oder weiteren Lösungsmitteln in an sich bekannter Weise mit Alkylierungsmitteln umsetzt.

Überraschenderweise wurde gefunden, daß sich vergleichsweise hydrophobe Fettalkoholpolyglycolether mit einem HLB-Wert im Bereich von 6 bis 12 in besonderer Weise als Lösungsmittel für die Quaternierung von sekundären bzw. tertiären Aminen eignen, so daß der Einsatz von Wasser, Alkoholen oder anderen organischen Lösungsmitteln an dieser Stelle vermieden wird. Gleichzeitig werden nach dem erfindungsgemäßen Verfahren erstmals vollständig wasserfreie Detergensmischungen mit einem hohen Gehalt an amphoteren bzw. zwitterionischen Tensiden zugänglich. Die Erfindung schließt ferner die im Hinblick auf die **EP-A2 0 353 580** nicht naheliegende Erkenntnis ein, daß die aus der Alkylierung resultierenden Betainverbindungen gerade mit den ausgewählten, vergleichsweise hydrophoben nichtionischen Tensiden praktisch unabhängig vom Molenbruch Mischmicellen, jedoch keine hochviskosen flüssigkristallinen Phasen bilden. Die wasserfreien Detergensgemische sind daher überraschenderweise niedrigviskos und stabil, d. h. es wird weder eine Phasentrennung, noch die Abscheidung von anorganischen Salzen beobachtet.

### Amine

Als Aminkomponenten kommen sekundäre, insbesondere tertiäre Amine in Betracht. Geeignete Ausgangsstoffe sind beispielsweise **Di-, vorzugsweise Trialkylamine** der Formel **(I)**, 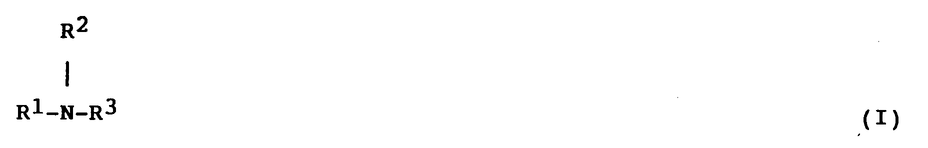 in der R¹ für Alkyl- und/oder Alkenylreste mit 6 bis 22 Kohlenstoffatomen, R² für Wasserstoff oder Alkylreste mit 1 bis 4 Kohlenstoffatomen und R³ für Alkylreste mit 1 bis 4 Kohlenstoffatomen steht.

Typische Beispiele sind Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische.

Als weitere Einsatzstoffe kommen auch **Amidoamine** der Formel **(II)** in Betracht, 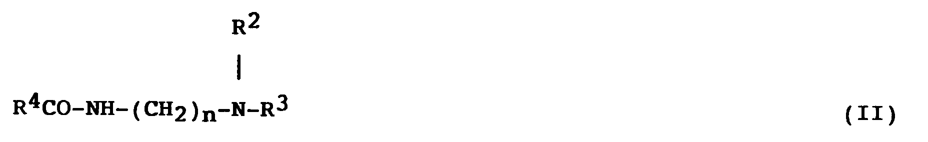 in der R⁴CO für einen aliphatischen Acylrest mit 6 bis 22 Kohlenstoffatomen und 0 oder 1 bis 3 Doppelbindungen, n für Zahlen von 1 bis 3 steht und R² und R³ die oben angegebenen Bedeutungen haben.

Amidoamine stellen bekannte Stoffe dar, die nach den einschlägigen Verfahren der präparativen Chemie erhalten werden können. Ein Verfahren zu ihrer Herstellung besteht beispielsweise darin, Fettsäuren mit Diaminen zu amidieren. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin. Bevorzugt ist der Einsatz von C_{12/14}-Kokosfettsäure-N,N-dimethylaminopropylamid.

Weiterhin kommen als geeignete Amine **Imidazoline** der Formel **(III)** in Betracht, 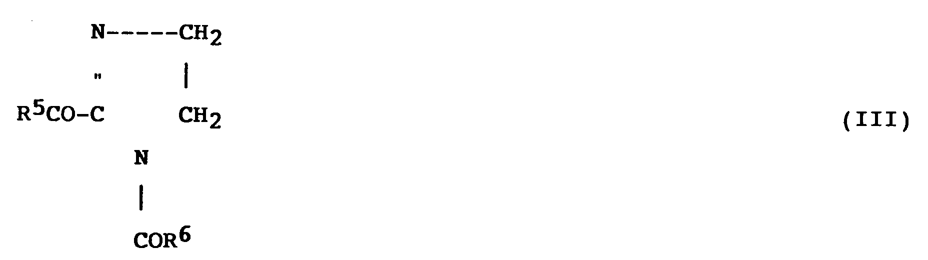 in der R^{5CO} und R⁶CO unabhängig voneinander für aliphatische Acylreste mit 6 bis 22 Kohlenstoffatomen und 0 oder 1 bis 3 Doppelbindungen stehen.

Auch bei diesen Substanzen handelt es sich um bekannte Stoffe, die beispielsweise durch cyclisierende Kondensation von 2 Mol Fettsäure mit Ethylendiamin erhalten werden können.

Typische Beispiele sind Kondensationsprodukte der oben genannten Fettsäuren mit Ethylendiamin, vorzugsweise Imidazoline auf Basis von Laurinsäure oder wiederum C_{12/14}-Kokosfettsäure.

### Fettalkoholpolyglycolether

Bei den im Sinne des erfindungsgemäßen Verfahrens einzusetzenden Fettalkoholpolyglycolether handelt es sich um vergleichseise hydrophobe Substanzen, die einen HLB-Wert im Bereich von 6 bis 12, vorzugsweise 8 bis 11 aufweisen.

Die Fettalkoholpolyglycolether folgen der Formel **(IV)**, 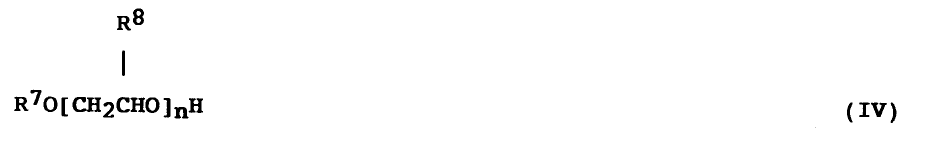 in der R⁷ für einen Alkylrest mit 8 bis 14 Kohlenstoffatomen, R⁸ für Wasserstoff oder eine Methylgruppe und n für Zahlen von 1 bis 10 steht.

Typische Beispiele stellen Anlagerungsprodukte von durchschnittlich 1 bis 10 Mol Ethylenoxid und/oder Propylenoxid an Caprylalkohol, Caprinalkohol, Laurylalkohol, Myristylalkohol oder insbesondere C_{12/14}-Kokosfettalkohol dar. Vorzugsweise werden Anlagerungsprodukte von durchschnittlich 0 bis 2 Mol Propylenoxid und 3 bis 8 Mol Ethylenoxid an Octanol oder C_{12/14}-Kokosfettalkohol eingesetzt. Die Addukte können eine konventionelle, breite Homologenverteilung und daher noch einen Restgehalt an freiem Fettalkohol aufweisen. In einer bevorzugten Ausführungsform der Erfindung werden jedoch Fettalkoholpolyglycolether mit einer eingeengten Homologenverteilung, sogenannte "narrow-range ethoxylates (NRE)", eingesetzt, die praktisch frei von Fettalkohol sind und in den erfindungsgemäßen Detergensgemischen eine besonders niedrige Endviskosität bewirken.

Die Amine und die Fettalkoholpolyglycolether können im molaren Verhältnis von 2 : 1 bis 1 : 2 eingesetzt werden. Im Hinblick auf die anwendungstechnischen Eigenschaften der resultierenden Detergensgemische hat sich jedoch ein molares Einsatzverhältnis von 1,8 : 1 bis 1 : 1,2 als optimal erwiesen.

### Alkylierungsmittel

Als Alkylierungsmittel können Halogencarbonsäuren bzw. deren Alkalisalze oder Ester eingesetzt werden. Aus Gründen der leichten Verfügbarkeit ist der Einsatz von Natriumchloracetat bevorzugt. Die Amine und die Halogencarbonsäuren bzw. deren Salze können im molaren Verhältnis von 1 : 1,0 bis 1 : 1,2, vorzugsweise 1 : 1 bis 1 : 1,15 eingesetzt werden.

### Quaternierung

Die Quaternierung bzw. Betainisierung der Amine kann in an sich bekannter Weise durchgeführt werden. Im Gegensatz zu den Verfahren des Stands der Technik ist die Anwesenheit eines weiteren Lösungsmittels, insbesondere Wasser, ausdrücklich nicht erforderlich und unerwünscht, da ansonsten keine wasserfreien Produkte erhalten würden. Üblicherweise wird die Alkylierung bei Temperaturen im Bereich von 70 bis 98°C durchführt und ist innerhalb von 1 bis 10, vorzugsweise 3 bis 7 h beendet.

Im Hinblick auf eine möglichst vollständige Abreaktion des Alkylierungsmittels hat es sich als vorteilhaft erwiesen, die Reaktion bei einem pH-Wert von 6 bis 10, vorzugsweise 7 bis 8,5 zu führen. Eine weitere Möglichkeit zur Minimierung des Restgehaltes an Alkylierungsmittel besteht ferner darin, der Reaktionsmischung nach Beendigung der Alkylierung einen definierten Überschuß an Aminosäuren, insbesondere Glycin zuzusetzen, wie dies in der **DE-A1 39 39 264** (Henkel) beschrieben wird.

Die Erfindung schließt als weitere Erkenntnis ein, daß die Abwesenheit von Wasser während der Alkylierung die als Konkurenzreaktion ablaufende Alkylierung des Polyglycolethers auf ein Minimum reduziert. Dementsprechend liegt der Gehalt an Ethercarbonsäuren in den nach dem erfindungsgemäßen Verfahren erhältlichen Produkten überraschenderweise sicher unter 1 Gew.-%.

### Wasserfreie Detergensgemische

Ein weiterer Gegenstand der Erfindung betrifft wasserfreie Detergensgemische, dadurch erhältlich, daß man sekundäre oder tertiäre Amine ohne Zusatz von Wasser oder organischen Lösungsmitteln, jedoch in Gegenwart von Fettalkoholpolyglycolethern mit einem HLB-Wert im Bereich von 6 bis 12 in an sich bekannter Weise mit Alkylierungsmitteln umsetzt.

Die erfindungsgemäßen Detergensgemische sind stabil, d. h., auch bei längerer Lagerung ist weder eine Entmischung noch eine Salzabscheidung zu beobachten. Ein weiterer Vorteil ist darin zu sehen, daß die Gemische gegenüber mikrobiellen Befall ausreichend stabilisiert sind, so daR der Zusatz typischer Konservierungsmittel nicht erforderlich ist. Dies macht die Compounds insbesondere für kosmetische Produkte interessant.

Die Detergensgemische besitzen eine ausgezeichnete Reinigungsleistung und ein gutes Schaumvermögen. Sie können als Compounds in den Handel gebracht und vor Ort zu einer Rezeptur verarbeitet oder auch direkt zusammen mit anderen Tensiden und Inhaltsstoffen konfektioniert werden.

### Compounds der Zusammensetzung

- 30 bis 70 Gew.-%: Betain,
- 20 bis 50 Gew.-%: Fettalkoholpolyglycolether und
- 0 bis 10 Gew.-%: Salz,
wie sie nach dem erfindungsgemäßen Verfahren erhältlich sind, erweisen sich als niedrig viskos, besitzen ein ausgezeichnetes Reinigungs- und Schaumvermögen auf, sind dermatologisch gut verträglich, biologisch leicht abbaubar und lassen sich durch Zusatz von maximal 15 Gew.-% Wasser - bezogen auf die Gesamtmischung - beispielsweise zu einem hervorragenden Handgeschirrspülmittel konfektionieren.

### Gewerbliche Anwendbarkeit

Die nach dem erfindungsgemäßen Verfahren erhältlichen Detergensgemische sind lagerstabil und niedrigviskos. Da sie als wasserfreie Produkte anfallen, nehmen sie wenig Raum bei Lagerung und Transport ein. Sie weisen ausgezeichnete Detergenseigenschaften und eine hohe dermatologische Verträglichkeit auf; zudem sind sie vollständig biologisch abbaubar.

Ein weiterer Gegenstand der Erfindung betrifft daher die Verwendung der erfindungsgemäßen wasserfreien Detergensgemische zur Herstellung oberflächenaktiver Mittel, als da sind: Wasch-, Spül- und Reinigungsmittel sowie Produkte zur Haar- und Körperpflege. Die Detergensgemische können als solche oder aber nach Verdünnung mit Wasser eingesetzt werden und dann in Mengen von 1 bis 90, vorzugsweise 10 bis 75 Gew.-% - bezogen auf die Mittel - enthalten sein.

Die folgenden Beispiele sollen den Gegenstand der Erfindung näher erläutern.

### Beispiele

### Beispiel 1:

### C_{12/14}-Kokosfettsäureamidopropyl-N-N-dimethylaminobetain/C_{12/14}-Kokosfettalkohol-8 EO (HLB-Wert: 11)

In einem 1-m³-Rührkessel mit Dampfheizung wurden 368 kg (665 mol) eines Anlagerungsproduktes von durchschnittlich 8 mol Ethylenoxid an einen technischen C_{12/14}-Kokosfettalkohol (Dehydol^{(R)} LS8, Henkel KGaA, Düsseldorf/FRG) vorgelegt, auf 85°C erhitzt und unter Rühren mit 158 kg (1350 mol) Natriumchloracetat versetzt. Innerhalb von 1 h wurden 409 kg (1196 mol) gehärtetes C_{12/14}-Kokosfettsäureamidopropyl-N,N-dimethylamin zugesetzt und die Mischung bei 90°C gehalten, wobei der pH-Wert auf 7 bis 8,5 eingestellt wurde. Nach Beendigung der Zugabe ließ man 5 h weiterreagieren, bis die Aminzahl den Wert 7 erreicht hatte und nicht weiter abnahm.

### Zusammensetzung der resultierenden Paste:

- 59 Gew.-%: Betain
- 39 Gew.-%: Fettalkoholpolyglycolether
- 2 Gew.-%: Salz

### Beispiel 2:

### C_{12/14}-Kokosfettsäureamidopropyl-N-N-dimethylaminobetain/C_{12/14}-Kokosfettalkohol-3 EO (HLB-Wert 8)

In einem 1-m³-Rührkessel mit Dampfheizung wurden 309 kg (928 mol) eines Anlagerungsproduktes von durchschnittlich 3 mol Ethylenoxid an einen technischen C_{12/14}-Kokosfettalkohol (Dehydol^{(R)} LS3, Henkel KGaA, Düsseldorf/FRG) vorgelegt, auf 85°C erhitzt und unter Rühren mit 108 kg (923 mol) Natriumchloracetat versetzt. Innerhalb von 1 h wurden 280 kg (819 mol) gehärtetes C_{12/14}-Kokosfettsäureamidopropyl-N,N-dimethylamin zugesetzt und die Mischung bei 90°C gehalten, wobei der pH-Wert auf 7 bis 8,5 eingestellt wurde. Nach Beendigung der Zugabe ließ man 6 h weiterreagieren, bis die Aminzahl den Wert 6,2 erreicht hatte und nicht weiter abnahm.

### Zusammensetzung der resultierenden Paste:

- 54 Gew.-%: Betain
- 44 Gew.-%: Fettalkoholpolyglycolether
- 2 Gew.-%: Salz

### Beispiel 3:

### C_{12/14}-Kokosfettsäureamidopropyl-N-N-dimethylaminobetain/C_{12/14}-Kokosfettalkohol-8 EO (HLB-Wert: 11)

In einem 1-m³-Rührkessel mit Dampfheizung wurden 523 kg (946 mol) eines Anlagerungsproduktes von durchschnittlich 8 mol Ethylenoxid an einen technischen C_{12/14}-Kokosfettalkohol (Dehydol^{(R)} LS3, Henkel KGaA, Düsseldorf/FRG) vorgelegt, auf 85°C erhitzt und unter Rühren mit 409 kg (1196 mol) gehärtetes C_{12/14}-Kokosfettsäureamidopropyl-N,N-dimethylamin versetzt. Innerhalb von 20 min wurden 143 kg (1222 mol) Natriumchloracetat zugesetzt und die Mischung bei 90°C gehalten, wobei der pH-Wert auf 7 bis 8,5 eingestellt wurde. Nach Beendigung der Zugabe ließ man 3 h weiterreagieren, bis die Aminzahl den Wert 6,3 erreicht hatte und nicht weiter abnahm.

### Zusammensetzung der resultierenden Paste:

- 51 Gew.-%: Betain
- 47 Gew.-%: Fettalkoholpolyglycolether
- 2 Gew.-%: Salz

### Beispiel 4:

### C_{12/14}-Kokosfettsäureamidopropyl-N-N-dimethylaminobetain/C₈-Kokosfettalkohol-1,5 PO,6 EO (HLB-Wert: 10,5)

In einem 1-m³-Rührkessel mit Dampfheizung wurden 448 kg (950 mol) eines Anlagerungsproduktes von durchschnittlich 1,5 mol Propylenoxid und 6 mol Ethylenoxid an Octanol vorgelegt, auf 85°C erhitzt und unter Rühren mit 409 kg (1196 mol) gehärtetes C_{12/14}-Kokosfettsäureamidopropyl-N,N-dimethylamin versetzt. Innerhalb von 20 min wurden 143 kg (1222 mol) Natriumchloracetat zugesetzt und die Mischung bei 90°C gehalten, wobei der pH-Wert auf 7 bis 8,5 eingestellt wurde. Nach Beendigung der Zugabe ließ man 3 h weiterreagieren, bis die Aminzahl den Wert 6,1 erreicht hatte und nicht weiter abnahm.

### Zusammensetzung der resultierenden Paste:

- 52 Gew.-%: Betain
- 46 Gew.-%: Fettalkoholpolyglycolether
- 2 Gew.-%: Salz

## Patentansprüche

1. Verfahren zur Herstellung wasserfreier Detergensgemische, bei dem man sekundäre oder tertiäre Amine in Gegenwart von Fettalkoholpolyglycolethern mit einem HLB-Wert im Bereich von 6 bis 12, jedoch ohne Zusatz von Wasser oder organischen Lösungsmitteln in an sich bekannter Weise mit Alkylierungsmitteln umsetzt.

2. Verfahren nach Anspruch 1, **dadurch gekennzeichnet,** daß man sekundäre oder tertiäre Amine der Formel **(I)** einsetzt, 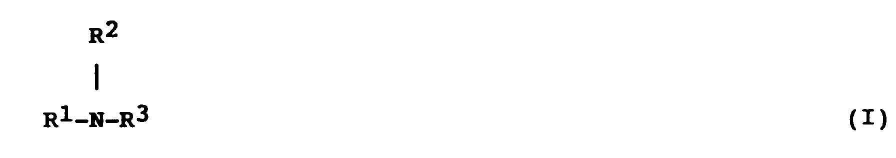 in der R¹ für Alkyl- und/oder Alkenylreste mit 6 bis 22 Kohlenstoffatomen, R² für Wasserstoff oder Alkylreste mit 1 bis 4 Kohlenstoffatomen und R³ für Alkylreste mit 1 bis 4 Kohlenstoffatomen steht.

3. Verfahren nach Anspruch 1, **dadurch gekennzeichnet,** daß man Amidoamine der Formel **(II)** einsetzt, 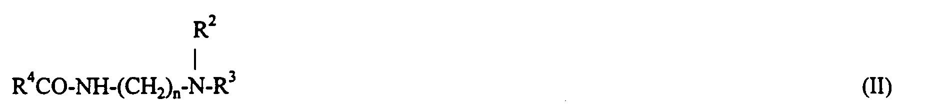 in der R⁴CO für einen aliphatischen Acylrest mit 6 bis 22 Kohlenstoffatomen und 0 oder 1 bis 3 Doppelbindungen, n für Zahlen von 1 bis 3 steht und R² und R³ die oben angegebenen Bedeutungen haben.

4. Verfahren nach Anspruch 1, **dadurch gekennzeichnet,** daß man Imidazoline der Formel **(III)** einsetzt, 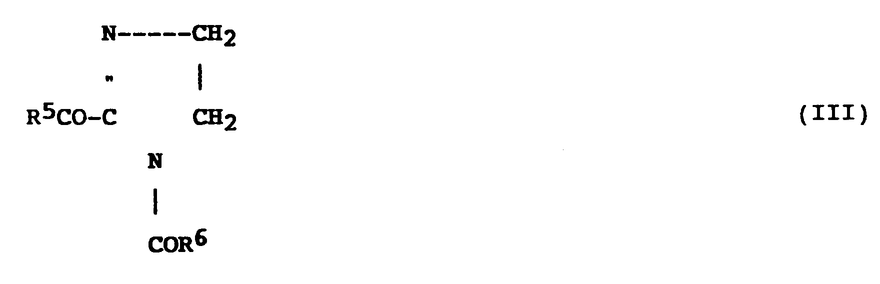 in der R^{5CO} und R⁶CO unabhängig voneinander für aliphatische Acylreste mit 6 bis 22 Kohlenstoffatomen und 0 oder 1 bis 3 Doppelbindungen stehen.

5. Verfahren nach den Ansprüchen 1 bis 4, **dadurch gekennzeichnet,** daß man Fettalkoholpolyglycolether mit einem HLB-Wert im Bereich von 8 bis 11 einsetzt.

6. Verfahren nach den Ansprüchen 1 bis 5, **dadurch gekennzeichnet,** daß man Fettalkoholpolyglycolether der Formel **(IV)** einsetzt, 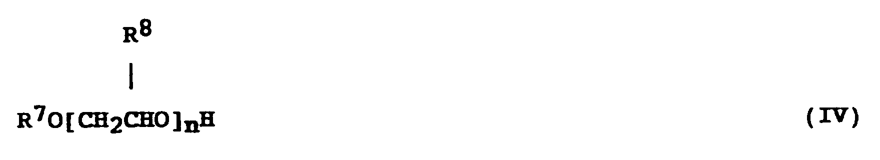 in der R⁷ für einen Alkylrest mit 8 bis 14 Kohlenstoffatomen, R⁸ für Wasserstoff oder eine Methylgruppe und n für Zahlen von 1 bis 10 steht.

7. Verfahren nach den Ansprüchen 1 bis 6, **dadurch gekennzeichnet,** daß man die Amine und die Fettalkoholpolyglycolether im molaren Verhältnis von 2 : 1 bis 1 : 2 einsetzt.

8. Verfahren nach den Ansprüchen 1 bis 7, **dadurch gekennzeichnet,** daß man als Alkylierungsmittel Halogencarbonsäuren bzw. deren Alkalisalze oder Ester einsetzt.

9. Verfahren nach den Ansprüchen 1 bis 8, **dadurch gekennzeichnet,** daß man die Amine und die Halogencarbonsäuren bzw. deren Salze im molaren Verhältnis von 1 : 1,0 bis 1 : 1,2 einsetzt.

10. Verfahren nach den Ansprüchen 1 bis 9, **dadurch gekennzeichnet,** daß man die Alkylierung bei Temperaturen im Bereich von 70 bis 98°C durchführt.

11. Verfahren nach den Ansprüchen 1 bis 10, **dadurch gekennzeichnet,** daß man die Alkylierung über einen Zeitraum von 1 bis 10 h durchführt.

12. Verfahren nach den Ansprüchen 1 bis 11, **dadurch gekennzeichnet,** daß man die Alkylierung bei einem pH-Wert von 6 bis 10 durchführt.

13. Wasserfreie Detergensgemische, dadurch erhältlich, daß man sekundäre oder tertiäre Amine in Gegenwart von Fettalkoholpolyglycolethern mit einem HLB-Wert im Bereich von 6 bis 12, jedoch ohne Zusatz von Wasser oder organischen Lösungsmitteln in an sich bekannter Weise mit Alkylierungsmitteln umsetzt.

14. Verwendung von wasserfreien Detergensgemischen nach dem Verfahren nach den Ansprüchen 1 bis 12 zur Herstellung oberflächenaktiver Mittel.

## Claims

1. A process for the production of water-free detergent mixtures, in which secondary or tertiary amines are reacted with alkylating agents in known manner in the presence of fatty alcohol polyglycol ethers with an HLB value of 6 to 12, but without any addition of water or organic solvents.

2. A process as claimed in claim 1, **characterized in that** secondary or tertiary amines corresponding to formula **(I):** 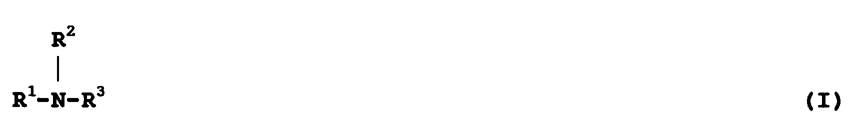 in which R¹ represents alkyl and/or alkenyl radicals containing 6 to 22 carbon atoms, R² represents hydrogen or alkyl radicals containing 1 to 4 carbon atoms and R³ represents alkyl radicals containing 1 to 4 carbon atoms, are used.

3. A process as claimed in claim 1, **characterized in that** amidoamines corresponding to formula **(II):** 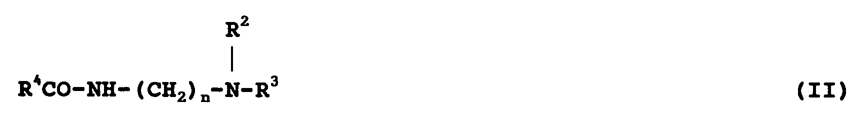 in which R⁴CO is an aliphatic acyl radical containing 6 to 22 carbon atoms and 0 or 1 to 3 double bonds, n is a number of 1 to 3 and R² and R³ are as defined above, are used.

4. A process as claimed in claim 1, **characterized in that** imidazolines corresponding to formula **(III):** 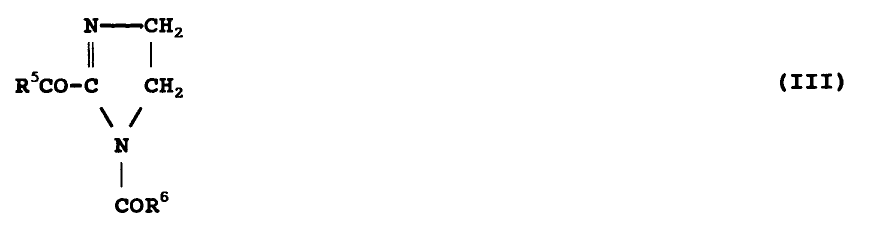 in which R⁵CO and R⁶CO independently of one another represent aliphatic acyl radicals containing 6 to 22 carbon atoms and 0 or 1 to 3 double bonds, are used.

5. A process as claimed in claims 1 to 4, **characterized in that** fatty alcohol polyglycol ethers with an HLB value of 8 to 11 are used.

6. A process as claimed in claims 1 to 5, **characterized in that** fatty alcohol polyglycol ethers corresponding to formula **(IV):** 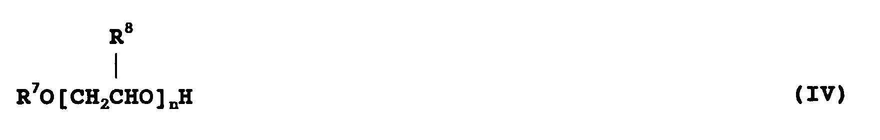 in which R⁷ is an alkyl radical containing 8 to 14 carbon atoms, R⁸ is hydrogen or a methyl group and n is a number of 1 to 10, are used.

7. A process as claimed in claims 1 to 6, **characterized in that** the amines and the fatty alcohol polyglycol ethers are used in a molar ratio of 2:1 to 1:2.

8. A process as claimed in claims 1 to 7, **characterized in that** halocarboxylic acids or alkali metal salts or esters thereof are used as the alkylating agent.

9. A process as claimed in claims 1 to 8, **characterized in that** the amines and the halocarboxylic acids or salts thereof are used in a molar ratio of 1:1.0 to 1:1.2.

10. A process as claimed in claims 1 to 9, **characterized** **in that** the alkylation is carried out at temperatures of 70 to 98°C.

11. A process as claimed in claims 1 to 10, **characterized in that** the alkylation is carried out over a period of 1 to 10 h.

12. A process as claimed in claims 1 to 11, **characterized in that** the alkylation is carried out at a pH value of 6 to 10.

13. Water-free detergent mixtures obtainable by reacting secondary or tertiary amines with alkylating agents in known manner in the presence of fatty alcohol polyglycol ethers with an HLB value of 6 to 12, but in the absence of water or organic solvents.

14. The use of the anhydrous detergent mixtures obtained by the process claimed in claims 1 to 12 for the production of surface-active compositions.

## Revendications

1. Procédé de fabrication de mélanges détergents anhydres, où des amines secondaires ou tertiaires sont transformées en présence de polyglycoléthers ayant un coefficient HLB compris entre 6 et 12, mais sans addition d'eau ou d'autres solvants organiques, de manière elle-même connue avec des agents d'alkylation.

2. Procédé selon la revendication 1,
caractérisé en ce que
l'on utilise des amines secondaires ou tertiaires de formule (I), 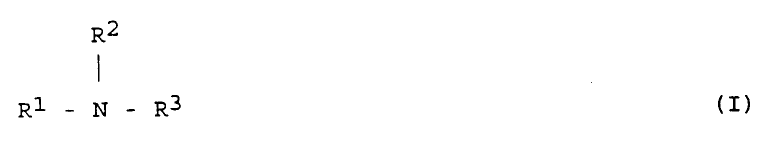 où R¹ représente un radical alkyle et/ou alkényle avec de 6 à 22 atomes de carbone, R² représente un hydrogène ou un radical alkyle avec de 1 à 4 atomes de carbone et R³ représente un radical alkyle avec de 1 à 4 atomes de carbone.

3. Procédé selon la revendication 1,
caractérisé en ce que
l'on utilise des amines de formule (II), 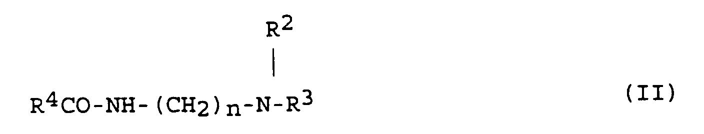 où R⁴CO représente un radical acyle aliphatique avec de 6 à 22 atomes de carbone et comportant 0 ou 1 à 3 doubles liaisons, et où n représente des nombres entre 1 et 3 et R² et R³ ont la signification indiquée ci-dessus.

4. Procédé selon la revendication 1,
caractérisé en ce que
l'on utilise des imidazolines de formule (III), 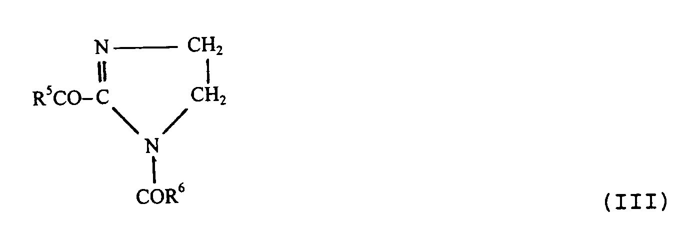 où R⁵CO et R⁶CO représentent, indépendamment entre eux, des radicaux acyle aliphatiques avec entre 6 et 22 atomes de carbone et comportant 0 ou 1 à 3 doubles liaisons.

5. Procédé selon les revendication 1 à 4,
caractérisé en ce que
l'on utilise des polyglycoléthers d'alcools gras ayant un coefficient HLB compris entre 8 et 11.

6. Procédé selon les revendications 1 à 5,
caractérisé en ce que
l'on utilise des polyglycoléthers d'alcools gras de formule (IV), 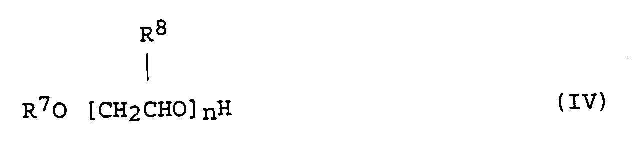 où R⁷ représente un radical aliphatique avec entre 8 et 14 atomes de carbone, R⁸ représente un hydrogène ou un groupe méthyle et où n représente un nombre de 1 à 10.

7. Procédé selon les revendications 1 à 6,
caractérisé en ce que
les amines et les polyglycoléthers d'alcools gras sont utilisés en proportion molaire de 2 : 1 à 1 : 2.

8. Procédé selon les revendications 1 à 7,
caractérisé en ce que
l'on utilise en tant qu'agent d'alkylation des acides carboxyliques halogénés ou leurs sels alcalins ou leurs esters.

9. Procédé selon les revendications 1 à 8,
caractérisé en ce que
l'on utilise les amines et les acides carboxyliques halogénés ou leurs sels dans une proportion molaire de 1 : 1,0 à 1 : 1,2.

10. Procédé selon les revendications 1 à 9,
caractérisé en ce que l'on réalise l'alkylation à des températures comprises entre 70 et 98°C.

11. Procédé selon les revendications 1 à 10,
caractérisé en ce que
l'on réalise l'alkylation pendant une durée de 1 à 10 h.

12. Procédé selon les revendications 1 à 11,
caractérisé en ce que
l'on réalise l'alkylation à un pH de 6 à 10.

13. Mélanges détergents anhydres, obtenus en ce que l'on laisse réagir des amines secondaires ou tertiaires avec des agents d'alkylation de manière elle-même connue, en présence de polyglycoléthers d'alcools gras ayant un coefficient HLB compris entre 6 et 12, mais sans addition d'eau ou d'autres solvants organiques.

14. Utilisation de mélanges détergent anhydres d'après le procédé selon les revendication 1 à 12 pour la fabrication de produits surfactants.
